Europäisches Patentamt

European Patent Office

Office européen des brevets

(19)

(11) Veröffentlichungsnummer: **0 044 041**
**B1**

(12) **EUROPÄISCHE PATENTSCHRIFT**

(45) Veröffentlichungstag der Patentschrift:
20.03.85

(51) Int. Cl.⁴: **G 01 N 33/539**

(21) Anmeldenummer: **81105350.3**

(22) Anmeldetag: **09.07.81**

(54) Verfahren zum Binden und Trennen für den kompetitiven Radioimmunoassay und Reagenz hierzu.

(30) Priorität: **14.07.80 DE 3026665**

(43) Veröffentlichungstag der Anmeldung:
**20.01.82 Patentblatt 82/3**

(45) Bekanntmachung des Hinweises auf die Patenterteilung:
**20.03.85 Patentblatt 85/12**

(84) Benannte Vertragsstaaten:
**AT BE CH DE FR GB IT LI LU NL SE**

(56) Entgegenhaltungen:
DE - A - 2 539 242
DE - A - 2 608 667
DE - A - 3 030 806
US - A - 4 018 883
US - A - 4 139 604

CHEMICAL ABSTRACTS, Band 90, Nr. 21, 21. Mai 1979, Seite 415, Spalte 1, Nr. 166291d Columbus, Ohio, U.S.A. P.G. NATALI et al.: "Antibody-coated protein A-bearing Staphylococcus aureus: a versatile and stable immune reagent"
CLINICAL CHEMISTRY, Band 26, Nr. 1, Januar 1980, Seiten 37-40, Pennsylvania, U.S.A. J. GAULDIE et al.: "Solid-phase radioimmunoassay with Protein-A-bearing staphylococcus aureus cells used to assay a protein (Ferritin) and a hapten (digoxin)"

(73) Patentinhaber: **HOECHST AKTIENGESELLSCHAFT,
Postfach 80 03 20, D-6230 Frankfurt am Main 80 (DE)**

(72) Erfinder: **Hachmann, Henning, Dr., Merziger Weg 1b,
D-6000 Frankfurt am Main 71 (DE)**
Erfinder: **Strecker, Helmut, Dr., Odenwaldstrasse 56,
D-6102 Pfungstadt (DE)**
Erfinder: **Seidel, Lothar, Dr., Gundelhardtstrasse 2,
D-6233 Kelkheim (Taunus) (DE)**

(56) Entgegenhaltungen: (Fortsetzung)
CLINICAL CHEMISTRY, Band 25, Nr. 5, Mai 1979, Seiten 752-756, Pennsylvania, U.S.A. R.K. GUPTA et al.: "Double-antibody method and the protein-A-bearing staphylococcus aureus cells method compared for separating bound and free antigen in radioimmunoassay"
BIOCHIMIE, Band 58, Nr. 11-12, 1976, Seiten 1417-1418 H. KERCRET et al.: "Utilisation du polyéthylène glycol pour la précipitation du complexe hormone-anticorps dans le dosage radioimmunologique des gonadotrophines de rat"
Fundamentals of Clinical Chemistry, Ed. N.W. Tietz (1976) pp. 286-290
Journal of Immunological Methods, 25 (1979) pp. 255-264

**Beschreibung**

Beim kompetitiven Radioimmunoassay wird ein radioaktiv markiertes Antigen, ein unmarkiertes Antigen und ein für das Antigen spezifischer Antikörper einer kompetitiven Antigen-Antikörper-Reaktion in einer wässrigen Pufferlösung unterworfen. Dabei konkurrieren eine definierte Menge an radioaktiv markiertem Antigen und unmarkiertes Antigen um eine definierte Menge Antikörper, wobei der Antikörper im Vergleich zum Antigen im Unterschuss vorliegt. Der am Antikörper gebundene Anteil des radioaktiv markierten Antigens ist von der Menge des unmarkierten Antigens abhängig und nimmt mit steigender Menge des unmarkierten Antigens ab.

Das Prinzip der radioimmunometrischen Messmethode beruht darauf, dass der am Antikörper gebundene Anteil des radioaktiv markierten Antigens, der bei einer zu messenden Probe beobachtet wird, verglichen wird mit dem Ergebnis von Eichproben mit bekanntem Antigengehalt und dadurch der Antigengehalt der zu messenden Probe bestimmt wird. Für die Messung des am Antikörper gebundenen Anteils des radioaktiv markierten Antigens ist es notwendig, den Antigen-Antikörper-Komplex vom nicht gebundenen Antigen abzutrennen. Die Methode des kompetitiven Radioimmunoassay ist im einzelnen in der Literatur beschrieben (vgl. R.P. Ekins, Br. Med. Bull. 30, 3–11 (1974), sowie C.M. Boyd, D.L. Herzberg, in: Practical radioimmunoassay S. 1–13 (A.J. Moss, G.V. Dalrymple, C.M. Boyd, eds.) The C.V. Mosby Company, Saint Louis 1976).

Die Genauigkeit des kompetitiven Radioimmunoassays hängt wesentlich von der Trennung des an den Antikörper gebundenen radioaktiv markierten Antigens vom freien radioaktiv markierten Antigen nach Beendigung der Antigen-Antikörper-Reaktion ab.

Methoden und Trennreagenzien zur Trennung von Antikörpergebundenen und freien Antigenen sind bekannt, z.B.

1. Fällung des Antigen-Antikörper-Komplexes mit Proteinfällungsreagenzien, vgl. B. Desbuquois, G.D. Aurbach J. Clin. Endocr. 33, 732–738 (1971).

2. Adsorption des freien Antigens an Adsorptionsmittel.

3. Bindung des Antikörpers (vor der Antigen-Antikörper-Reaktion) an Oberflächen von wasserunlöslichen Festkörpern, vgl. DOS 2 322 533.

4. Adsorption des Antigen-Antikörper-Komplexes an Staphylococcus aureus-Zellen, vgl. S. Jonsson, G. Kronvall Eur. J. Immunol. 4, 29–33 (1974).

5. Fällung des Antigen-Antikörper-Komplexes durch einen zweiten Antikörper (Trennantikörper), vgl. M.J. Martin, J. Landon Radioimmunoassay in Clinical Biochemistry S. 269–281 (Pasternak, editor) Heyden, London (1975).

6. Adsorption des Antigen-Antikörper-Komplexes an kleinen, wasserunlöslichen Partikeln (Festkörpern), die mit einem zweiten Antikörper (Trennantikörper) beschichtet sind, vgl. F.C. Den Hollander, A.H. W.M. Schuurs Radioimmunoassay Methods S. 419–422 (Kirkham and Hunter, eds.) Churchill Livingstone, London (1971); S. Jonsson, G. Kronvall IAEA Report SM 177/48, 287–298 (1974).

Die Trennung von Antikörper-gebundenem und freiem Antigen wird durch eine Phasentrennung vorgenommen, der meist ein Zentrifugationsschritt vorausgeht.

Die oben genannten Methoden können wie folgt beschrieben werden:

1. Die Fällung von Antigen-Antikörper-Komplexen durch Proteinfällungsreagenzien wie Ethanol, Ammonsulfat oder Polyethylenglykol ist einfach durchzuführen, da sofort nach Vermischen mit dem Fällungsreagenz eine Trennung durch Zentrifugieren möglich ist. Diese Methode setzt jedoch voraus, dass in der zu messenden Probe eine ausreichende Menge an Immunoglobulinen enthalten ist. Dies ist zwar in Serum oder Plasma, nicht aber in Urin, Liquor oder Magensaft der Fall.

Effektive Trennungen sind zudem nur bei Antigenen mit einem Molgewicht möglich, das wesentlich unter dem Molgewicht der Antikörper liegt. Dementsprechend ist dieses Verfahren nur für Spezialfälle anwendbar. Ferner hat es den Nachteil, dass ein oftmals nicht unwesentlicher Anteil des freien Antigens mitgefällt wird. Diese Mitfällung schwankt mit dem Proteingehalt und der Proteinzusammensetzung der Probe und kann das Messergebnis stören.

2. Die Adsorption des freien Antigens an Adsorptionsmittel wie Aktivkohle, Ionenaustauscher und Silikate ist nur für kleine Antigene (z.B. Steroide, Peptide) geeignet und nicht allgemein anwendbar. Zudem kann das Reaktionsgleichgewicht bei längerer Reaktionszeit mit dem Adsorptionsmittel gestört werden.

3. Bei Anwendung eines Antikörpers, der an Oberflächen von wasserunlöslichen Festkörpern (z.B. Teströhrcheninnenwand, Papierstreifen, Polymerkugeln verschiedener Grössen, Glasperlen, Bakterienzellen) gebunden ist, liegt der während der Inkubation gebildete Antigen-Antikörper-Komplex ebenfalls am Festkörper gebunden vor und wird mit dem Festkörper vom freien Antigen, das in der Reaktionslösung enthalten ist, abgetrennt. Die jeweils gleiche Menge an Antikörper, mit der solche Festkörper beschichtet sind, ist Voraussetzung für die Genauigkeit des kompetitiven Radioimmunoassays und ist nur mit grossem Aufwand und Sorgfalt erreichbar. Zusätzlich treten gelegentlich unerwünschte Störungen auf durch Adsorption von Proteinen aus der Probe an dem Festkörper und der damit verbundenen Blockierung der Antikörper, die in definierter Menge wirksam sein müssen.

4. Aufgrund der spezifischen Adsorptionseigenschaften der Staphylococcus aureus-Zellen für einige Klassen von Immunglobulinen verschiedener Tierspezies – die beim Radioimmunoassay verwendeten Antikörper zählen zu den Immunglobulinen – können Staphylococcus aureus-Zellen als Adsorbens für Antiben-Antikörper-Komplexe dienen. Die Methode ist nur anwendbar, wenn der Antikörper einer Immunglobulinklasse von einer Tierspezies angehört, deren entsprechendes Immunglobulin vom Staphylococcus aureus spezifisch gebunden sind. Dementsprechend ist das Verfahren nicht für Antikörper jeder beliebigen Tierspezies anwendbar.

Enthält neben dem als Reagenz verwendeten Antikörper zusätzlich auch die zu messende Probe (z.B. Serum oder Plasma) grössere Mengen von Staphylococcus aureus bindendem Immunglobulin, so muss eine entsprechend grössere Menge an Staphylococcus aureus-Zellen eingesetzt werden. Dies hat den Nachteil, dass durch unspezifische Adsorptionen von freiem Antigen die Trennung nicht vollständig ist.

5. Die Fällung des Antigen-Antikörper-Komplexes durch einen zweiten Antikörper (Trennantikörper), der gegen den Antigen-spezifischen ersten Antikörper gerichtet ist, ist zeitraubend, weil zwei Antigen-Antikörper-Reaktionen durchgeführt werden müssen. Die zweite Antigen-Antikörper-Reaktion kann durch Reaktionsbeschleuniger abgekürzt werden. Diese Methode hat den Nachteil, dass unerwünschte Mitfällungen von freiem Antigen auftreten, die vom schwankenden Proteingehalt der Probe bestimmt werden. Zudem ist der Niederschlag nach der Fällung schlecht sichtbar und kann beim Abtrennen des Überstandes unbemerkt verloren gehen.

6. Die Adsorption des Antigen-Antikörper-Komplexes durch einen zweiten Antikörper (Trennantikörper), der gegen den Antigen-spezifischen ersten Antikörper gerichtet ist und kovalent an einem wasserunlöslichen Festkörper (z.B. Cellulose, Staphylococcus aureus) gebunden ist, hat den Nachteil, dass zur vollständigen Adsorption des Antigen-Antikörper-Komplexes einige Stunden benötigt werden, während denen zudem durch ständige Bewegung des Reaktionsgefässes eine homogene Suspension des Festkörpers erzeugt werden muss.

Die Aufgabe der vorliegenden Erfindung ist die Bereitstellung eines Trennverfahrens und eines Trennreagenzes zur Trennung von freiem Antigen von einem an einem ersten Antikörper gebundenen Antigen beim kompetitiven Radioimmunoassay, wobei das Trennverfahren und das Trennreagenz die Nachteile der oben unter 1 bis 6 genannten Trennverfahren und Trennreagenzien vermeidet.

Die Erfindung betrifft ein Verfahren zum Binden eines an einen ersten Antikörper spezifisch gebundenen Antigens in einer wässrigen Lösung und zum Abtrennen dieses gebundenen Antigens von nicht gebundenem Antigen zur qualitativen oder quantitativen Bestimmung der Antigenkonzentration in einer Körperflüssigkeit mit Hilfe des kompetitiven Radioimmunoassays, wobei der erste Antikörper mit dem spezifisch gebundenen Antigen durch eine zweiten Antikörper, der einerseits an einer Staphylococcus aureus-Zelle gebunden ist und anderseits den ersten Antikörper spezifisch bindet, von dem freien, in der wässrigen Lösung gelösten Antigen abgetrennt wird, dadurch gekennzeichnet, dass der wässrigen Lösung ein Reaktionsbeschleuniger zugesetzt wird, der die spezifische Bindung des ersten Antikörpers durch den zweiten Antikörper beschleunigt, wobei als Reaktionsbeschleuniger 4–8 Gewichtsprozent Polyethylenglykol, 5–10 Gewichtsprozent Dextran, 5–10 Gewichtsprozent Polyvinylpyrrolidon, 0,5–0,9 molar Ammoniumsulfat oder 0,8–1,2 molar Natriumsulfit verwendet wird, so dass die Bindungsreaktion in wenigen Minuten abgeschlossen ist.

Die im Verfahren gemäss der Erfindung verwendeten Antigene sind Peptide, Proteine oder Glykoproteine, die ihrer Funktion entsprechend Hormone, Enzyme, Transportproteine oder Strukturproteine sein können und die bei der Immunisierung verschiedener Wirbeltierarten, vorzugsweise Säugetieren, eine Bildung von Antigenspezifischen ersten Antikörpern hervorrufen.

Die Bestimmung des Antigens in Körperflüssigkeiten wie Blutplasma, Blutserum, Urin, Liquor, Magensaft, Duodenalsaft, Fruchtwasser oder Synovialflüssigkeit wird mit Hilfe des kompetitiven Radioimmunoassays vorgenommen, wobei die genannten Körperflüssigkeiten vom Menschen oder von einem Tier stammen können.

Als zweite Antikörper finden solche verschiedener Tierklassen, in erster Linie von Wirbeltieren, vorzugsweise von Säugetieren Verwendung, wobei dieser zweite Antikörper durch Immunisierung der Tiere mit Immunglobulinen einer fremden Tierart, von der der erste Antikörper stammt, gewonnen wurde. Staphylococcus aureus-Zellen binden Gamma-Globuline vom Menschen oder von verschiedenen Tierarten wie Esel, Ziege, Schwein, Hund, Katze, Meerschweinchen oder Kaninchen spezifisch über ihr Fc-Fragment, wobei das Antigen-spezifische Fab-Fragment der Gamma-Globuline von der Bindung an der Staphylococcus aureus-Zelle unberührt bleibt (vgl. J.W. Goding, J. Immunol. Meth. 20, 241–253 (1978)).

Vorteilhaft ist die Verwendung eines zweiten Antikörpers von grösseren Tieren wie Ziege oder Esel. Der zweite Antikörper lässt sich durch Inkubation der Staphylococcus aureus-Zelle mit dem Serum des immunisierten Tieres binden, wobei insbesondere die Gamma-Globuline spezifisch gebunden werden und die nicht bindenden Serumproteine und anderen Serumbestandteile durch Waschen der Zellen leicht abgetrennt werden können, ohne dass eine wesentliche Menge des zweiten Antikörpers wieder desorbiert wird.

Für die Bestimmung eines Antigens in Körperflüssigkeiten, die grössere Mengen an Immunglobulinen, insbesondere Gamma-Globuline enthalten, wie Blutserum oder Blutplasma, ist es vorteilhaft, den zweiten Antikörper zusätzlich zu der genannten spezifischen (adsorptiven) Bindung kovalent an die Staphylococcus aureus-Zelle zu binden. Dazu wird der zweite Antikörper nach der spezifischen Adsorption an die Staphylococcus aureus-Zelle unter milden Bedingungen mittels eines bifunktionellen Reagenzes kovalent gebunden, wobei die Reaktionsfähigkeit des zweiten Antikörpers nicht wesentlich nachlässt. Aufgrund der kovalenten Bindung kann sich der zweite Antikörper nicht von der Zelle lösen und nicht durch Gamma-Globuline der Probe verdrängt werden.

Durch kovalente Bindung mit einem bifunktionellen Reagenz werden einerseits Aminogruppen des zweiten Antikörpers und anderseits Aminogruppen von Zellwandproteinen des Staphylococcus aureus mit einander verbunden. Als bifunktionelle Reagenzien werden Dialdehyde, Diimidate, Diester oder Diisocyanate, z.B. Glutardialdehyd, Dimethyladipindiimidat, Suberinsäuredimethylester oder Hexamethylendiisocyanat verwendet.

Die Staphylococcus aureus-Zellen werden vor Verwendung im Verfahren gemäss der Erfindung zweckmässig abgetötet durch Hitze- oder Formaldehydbehandlung. Vorteilhaft werden die Zellen dreissig Minuten auf 80 °C erwärmt und in einer 1.5%igen Formaldehydlösung inkubiert, wodurch die Bindungsfähigkeit der Zellen für Gamma-Globuline nicht wesentlich abnimmt.

Reaktionsbeschleuniger gemäss der Erfindung sind die hydrophilen Polymeren Dextran und Polyvinylpyrrolidon, die die spezifische Bindungsreaktion eines ersten Antikörpers durch einen zweiten Antikörper beschleunigen, wobei sie die räumliche Polypeptidstruktur der Immunoglobuline, insbesondere der Gamma-Globuline, nicht wesentlich verändern und damit die spezifische Reaktionsfähigkeit der Antikörper nicht zerstören. Vorteilhaft werden Polyethylenglykol, Ammoniumsulfat und Natriumsulfit verwendet, die in höheren Konzentrationen bevorzugt Immunglobuline, insbesondere Gamma-Globuline unlöslich machen oder ausfällen. Hierbei ist eine niedrigere Konzentration dieser Substanzen zu verwenden als sie zum Fällen des ersten Antikörpers und des Antigens führen würde.

Die Konzentration der Reaktionsbeschleuniger im Reaktionsgemisch bestehend aus dem ersten Antikörper und dem Antigen sowie dem an der Staphylococcus aureus-Zelle gebundenen zweiten Antikörper wird so gewählt, dass

– die Polyethylenglykolkonzentration vier bis acht Gewichts-%, im besonderen 5–6 Gewichts-% beträgt bzw.

– die Dextrankonzentration fünf bis zehn Gewichts-%, im besonderen sieben Gewichts-% beträgt bzw.

– die Polyvinylpyrrolidonkonzentration fünf bis zehn Gewichts-%, im besonderen sieben Gewichts-% beträgt bzw.

– die Ammoniumsulfatkonzentration 0.5–0.9 Molar, im besonderen 0.7–0.8 Molar ist bzw.

– die Natriumsulfitkonzentration 0.8–1.2 Molar, im besonderen 1.0 Molar ist.

Das Molekulargewicht der polymeren Reaktionsbeschleuniger liegt im Falle des Polyethylenglykols im allgemeinen zwischen 1000 und 20 000, vorzugsweise zwischen 2000 und 10 000, im besonderen bei 6000,

im Falle des Dextrans im allgemeinen zwischen 2000 und 50 000, vorzugsweise zwischen 4000 und 20 000, im besonderen zwischen 8000 und 12 000,

im Falle des Polyvinylpyrrolidons im allgemeinen zwischen 2000 und 25 000, vorzugsweise zwischen 5000 und 15 000, im besonderen bei 10 000.

Der Reaktionsbeschleuniger kann schon vor der Reaktion des ersten Antikörpers mit dem Antigen zum Reaktionsgemisch gegeben werden, bevor der am Staphylococcus aureus gebundene zweite Antikörper zur Trennung zugegeben wird. Vorteilhaft wird aber zum Reaktionsgemisch bestehend aus Antigen und erstem Antikörper ein gebrauchsfertiges Trennreagenz zugegeben.

Die Erfindung betrifft auch ein Reagenz gemäss Anspruch 2. Das gebrauchsfertige Trennreagenz enthält in einer wässrigen Pufferlösung den an Staphylococcus aureus gebundenen zweiten Antikörper sowie den Reaktionsbeschleuniger, dessen Konzentration so gewählt ist, dass nach der Zugabe des gebrauchsfertigen Trennreagenz zum Reaktionsgemisch bestehend aus Antigen und erstem Antikörper der Reaktionsbeschleuniger in den vorstehend genannten Konzentration vorliegt.

Die Menge des am Staphylococcus aureus gebundenen zweiten Antikörpers (bei Verwendung von 0.1 ml zu bestimmender Körperflüssigkeit) entspricht der Menge, die üblicherweise bei einer Fällung des Antigen-Antikörper-Komplexes durch einen nicht gebundenen zweiten Antikörper benötigt wird.

Die Menge an Staphylococcus beträgt (bei Verwendung von 0.1 ml zu bestimmender Körperflüssigkeit) im allgemeinen 0.5 bis 10 mg, vorzugsweise 1 bis 5 mg, im besonderen 2.5 mg.

Zur Durchführung der radioimmunometrischen Bestimmung werden zunächst radioaktiv markiertes Antigen, nicht markiertes Antigen und Antigenspezifischer erster Antikörper zusammen inkubiert. Dabei wird als nicht markiertes Antigen entweder eine Lösung von bekanntem Antigengehalt zur Verwendung als Eichprobe oder eine Körperflüssigkeit mit unbekanntem Antigengehalt als zu bestimmende Probe verwendet.

Die Inkubation wird im allgemeinen bei Temperaturen zwischen 0° und 45 °C, vorzugsweise zwischen 17° und 27 °C und gewöhnlich bei Raumtemperatur vorgenommen.

Das Trennreagenz wird vor der Zugabe zum Reaktionsgemisch geschüttelt, so dass eine gleichmässige Zellsuspension erhalten wird. Die Temperatur des Trennreagenz liegt im allgemeinen zwischen 0° und +45 °C, vorteilhaft wird sie der Temperatur der vorangehenden Inkubation angepasst.

Nach dem Vermischen des Trennreagenz mit dem Reaktionsgemisch ist die Bindung des ersten Antikörpers durch den zweiten Antikörper in wenigen Minuten abgeschlossen. Während dieser Zeit bleiben die Zellen wegen ihrer geringen Grösse gleichmässig in der Lösung verteilt, so dass sie nicht durch Sedimentation für eine Bindung verloren gehen. Durch anschliessende Zentrifugation wird das am ersten Antikörper gebundene Antigen mit der mit dem zweiten Antikörper beladenen Staphylococcus aureus-Zelle als Niederschlag von dem freien Antigen in der überstehenden Lösung abgetrennt.

Zur Bestimmung der Menge des am ersten Antikörper gebundenen markierten Antigens wird die überstehende Lösung dekantiert oder abgesaugt, wobei der Niederschlag zweckmässig anschliessend mit einer Pufferlösung gewaschen wird. Bei einem grösseren Volumen der überstehenden Flüssigkeit kann auf das Waschen verzichtet werden.

Die Bestimmung der Radioaktivität wird nach bekannten Verfahren durch Strahlungsdetektoren vorgenommen.

A. Das Trennverfahren gemäss der Erfindung ist bei der Bestimmung von Antigenen mit niedrigem oder hohem Molekulargewicht geeignet, insbesondere für Peptide und Proteine wie Follikelstimulierendes Hormon, luteinisierendes Hormon, Schilddrüsen-stimulierendes Hormon, Choriongondotropin, Prolaktin, Placentalaktogen, Wachstumshormon, adrenocortikotropes Hormon, Gukagon, β-Kette des Choriongonadotropin, Parathormon, Alphafetoprotein, carcinoembryonales Antigen, Prostata-spezifische saure Phosphate, schwangerschaftsspezifisches β,-Glykoprotein, die bei Abwesenheit Antigen-spezifischer Antikörper nicht an Staphylococcus aureus-Zellen gebunden werden.

B. Das Trennverfahren ist allgemein anwendbar zur Trennung von freiem Antigen und von an einem beliebigen ersten Antikörper gebundenen Antigen, wobei der erste Antikörper von einer beliebigen ersten Tierspezies stammt und der zweite Antikörper jeweils durch Immunisierung einer zweiten Tierspezies mit Immunglobulin der ersten Tierspezies gewonnen wird, wobei die zweite Tierspezies so gewählt wird, dass deren Gamma-Globuline grösstenteils von der Staphylococcus aureus-Zelle spezifisch gebunden werden.

C. Durch die spezifische Bindung des Antigenbeladenen ersten Antikörpers an den spezifisch an der Staphylococcus aureus-Zelle gebundenen zweiten Antikörper ergibt das Trennverfahren eine weitgehend quantitative Trennung des freien Antigens vom am ersten Antikörper gebundenen Antigen, die auch durch stark schwankenden Proteingehalt der zu bestimmenden Probe nicht wesentlich gestört wird.

D. Der Reaktionsbeschleuniger für die Bindung des Antigenbeladenen ersten Antikörpers durch den Staphylococcus aureus-gebundenen zweiten Antikörper erfordert nur eine sehr kurze zweite Inkubationszeit, während der ein ständiges Bewegen des Reaktionsgemisches zur homogenen Verteilung der Zellen überflüssig ist, so dass nach der Vermischung mit dem Trennreagenz ohne einen weiteren Arbeitsschritt und ohne grösseren Verzug zentrifugiert werden kann.

E. Der Zellniederschlag nach der Zentrifugation ist deutlich sichtbar und haftet gut am Boden des Zentrifugenröhrchens, so dass das Abtrennen der überstehenden Flüssigkeit durch Absaugen oder Dekantieren problemlos ist und visuell einfach kontrolliert werden kann.

Die Erfindung wird durch die folgenden Beispiele näher erläutert. Sämtliche Prozentangaben dort beziehen sich auf Gewichtsprozent.

Beispiel 1
Bestimmung des humanen luteinisierenden-Hormons (hLH) in Blutplasma oder Blutserum
a) Herstellung des Trennreagenzes

Zu 50 ml einer 10%igen Suspension von Staphylococcus aureus-Zellen in einer 0.9% Natriumchlorid enthaltenden 0.05 m wässrigen Natriumphosphatpufferlösung vom pH 7.4 wurden 16 ml eines Antiserums von der Ziege, das Antikörper gegen Gamma-Globulin vom Kaninchen enthält, gegeben. Die Suspension wurde eine Stunde gerührt. Anschliessend wurden die mit Antikörper beladenen Zellen durch Zentrifugieren und Dekantieren vom Überstand abgetrennt. Die Zellen wurden in 45 ml des oben genannten Puffers resuspendiert und zur kovalenten Bindung des Antikörpers an den Zellen unter ständigem Rühren mit 50 ml einer 0.2%igen Glutardialdehydlösung in dem oben genannten Puffer und nach einstündigem Rühren mit 50 ml einer 5%igen Natriumsulfitlösung in dem oben genannten Puffer versetzt, um den überschüssigen nicht umgesetzten Glutardialdehyd zu inaktivieren. Nach einer weiteren Stunde Rühren wurden die Zellen durch Zentrifugieren und Dekantieren vom Überstand abgetrennt. Die Zellen wurden gewaschen, indem sie in 200 ml 0.05 m wässrigen Natriumphosphatpuffer vom pH 7.4 resuspendiert wurden und durch Zentrifugieren und Dekantieren vom Waschpuffer abgetrennt wurden. Das gebrauchsfertige Trennreagenz wurde hergestellt, indem die so behandelten Zellen mit dem zweiten Antikörper anschliessend in 500 ml einer 10%igen Lösung von Polyethylenglykol mit einem Molekulargewicht von 6000 in 0.05 m wässrigen Natriumphosphatpuffer vom pH 7.4 resuspendiert wurden.

b) Durchführung der Bestimmung
Die Analysen wurden in Zentrifugenröhrchen von 3 bis 5 ml aus Polystyrol oder Polypropylen durchgeführt.

0.1 ml der zu untersuchenden humanen Serum- oder Plasmaprobe wurde in ein Röhrchen gegeben.

0.1 ml einer Eichprobe mit verschiedenen hLH-Konzentrationen, z.B. 0, 4, 7, 14, 27, 54 und 100 m Einheiten hLH/ml wurden jeweils in ein Röhrchen gegeben.

0.2 ml einer $^{125}$J-hLH enthaltenden Lösung (etwa 0.5 ng hLH/ml von einer spezifischen Radioaktivität von 100 nCi Jod-125/ng hLH) wurden in alle Röhrchen gegeben.

0.2 ml einer Antikörperlösung gegen hLH, die in Abwesenheit von unmarkiertem hLH 30 bis 50% des $^{125}$J-hLH binden konnte, wurde in alle Röhrchen gegeben.

Die Inkubation fand während zwanzig Stunden bei Raumtemperatur statt.

0.5 ml des gebrauchsfertigen Trennreagenz entsprechend Beispiel 1a), das vor Gebrauch geschüttelt wurde, um eine homogene Zellsuspension zu erhalten, wurde in alle Röhrchen gegeben.

Alle Röhrchen wurden zum Vermischen des Inhalts geschüttelt und nach mindestens zehn Minuten bei etwa 2000 g zehn Minuten lang zentrifugiert.

Der Niederschlag wurde durch Dekantieren oder Absaugen der überstehenden Flüssigkeit abgetrennt und mit einer 0.05 m wässrigen Natriumphosphatpufferlösung vom pH 7.4 gewaschen.

Nach dem Abtrennen der überstehenden Flüssigkeit wurde die Gamma-Strahlung des im Niederschlag enthaltenen markierten hLH in allen Röhrchen gemessen.

In einem Diagramm wurden die Impulse der Gamma-Strahlung der Eichproben pro Zeiteinheit linear gegen die hLH-Konzentration der Eichproben aufgetragen. Aus diesem Diagramm wurde dann die hLH-Konzentration der unbekannten Probe bestimmt.

Beispiel 2
Bestimmung des humanen Alphafetoproteins (AFP) in Fruchtwasser
a) Herstellung des Trennreagenzes

Zu 50 ml einer 10%igen Suspension von Staphylococcus aureus-Zellen in einer 0.9%iger wässriger Natriumchlorid wurden 50 ml eines Antiserums vom Esel, das Antikörper gegen Gamma-Globulin vom Kaninchen enthält, gegeben. Die Suspension wurde eine Stunde gerührt. Anschliessend wurden die Zellen durch Zentrifugieren und Dekantieren vom Überstand abgetrennt. Die Zellen wurden gewaschen, indem sie mit 200 ml 0.9%iger wässriger Natriumchloridlösung resuspendiert und durch Zentrifugieren und Dekantieren von der Waschlösung abgetrennt wurden.

Das gebrauchsfertige Trennreagenz wurde hergestellt, indem die so behandelten Zellen mit dem zweiten Antikörper anschliessend in 500 ml einer wässrigen Pufferlösung vom pH 7.4, die 1.6 m Natriumsulfit und 0.05 m Natriumphosphat enthielt, resuspendiert wurden.

b) Durchführung der Bestimmung

Die Analysen wurden in Zentrifugenröhrchen von 3 bis 5 ml aus Polystyrol oder Polypropylen durchgeführt.

0.1 ml der zu untersuchenden, zuvor 1 : 100 verdünnten Fruchtwasserprobe wurde in ein Röhrchen gegeben.

0.1 ml einer Eichprobe mit verschiedenen AFP-Konzentrationen, z.B. 0, 25, 50, 100, 200 und 400 Einheiten AFP/ml wurden jeweils in ein Röhrchen gegeben.

0.2 ml einer [125]J-AFP enthaltenden Lösung (etwa 1 Einheit AFP/ml von einer spezifischen Radioaktivität von 40 nCi Jod-125/Einheit AFP) wurden in alle Röhrchen gegeben.

0.2 ml einer Antikörperlösung gegen AFP, die in Abwesenheit von unmarkiertem AFP 40 bis 60% des [125]J-AFP binden konnte, wurde in alle Röhrchen gegeben.

Die Inkubation fand während zwanzig Stunden bei Raumtemperatur statt.

0.5 ml des gebrauchsfertigen Trennreagenz entsprechend Beispiel 2a), das vor Gebrauch kurz geschüttelt wurde, um eine homogene Zellsuspension zu erhalten, wurde in alle Röhrchen gegeben.

Alle Röhrchen wurden zum Vermischen des Inhalts kurz geschüttelt und fünf bis zehn Minuten später bei etwa 2000 g zehn Minuten lang zentrifugiert.

Der Niederschlag wurde durch Dekantieren oder Absaugen der überstehenden Flüssigkeit abgetrennt.

Nach dem Abtrennen der überstehenden Flüssigkeit wurde die Gamma-Strahlung des im Niederschlag enthaltenen markierten AFP in allen Röhrchen gemessen.

In einem Diagramm wurden die Impulse der Gamma-Strahlung der Eichproben pro Zeiteinheit linear gegen die AFP-Konzentration der Eichproben aufgetragen. Aus diesem Diagramm konnte dann die AFP-Konzentration der unbekannten Probe bestimmt werden.

Beispiel 3
Bestimmung des humanen Alphafetoproteins (AFP) in Fruchtwasser
a) Herstellung des Trennreagenzes

Die Herstellung des Trennreagenzes wurde einschliesslich des Waschschrittes entsprechend Beispiel 1a) durchgeführt.

Das gebrauchsfertige Trennreagenz wurde hergestellt indem die so behandelten Zellen mit dem zweiten Antikörper anschliessend in 500 ml einer 10%igen Lösung von Polyvinylpyrrolidon mit einem mittleren Molekulargewicht von 10 000 in 0.05 m wässrigem Natriumphosphatpuffer vom pH 7.4 resuspendiert wurden.

b) Durchführung der Bestimmung

Die Analysen wurden in Zentrifugenröhrchen von 3 bis 5 ml aus Polystyrol oder Polypropylen durchgeführt.

0.1 ml der zu untersuchenden, zuvor 1 : 100 verdünnten Fruchtwasserprobe wurde in ein Röhrchen gegeben.

0.1 ml einer Eichprobe mit verschiedenen AFP-Konzentrationen, z.B. 0, 25, 50, 100, 200 und 400 Einheiten AFP/ml wurden jeweils in ein Röhrchen gegeben.

0.2 ml einer [125]J-AFP enthaltenden Lösung (etwa 1 Einheit AFP/ml von einer spezifischen Radioaktivität von 40 nCi Jod-125/Einheit AFP) wurden in alle Röhrchen gegeben.

0.2 ml einer Antikörperlösung gegen AFP, die in Abwesenheit von unmarkiertem AFP 40 bis 60% des [125]J-AFP binden konnte, wurde in alle Röhrchen gegeben.

Die Inkubation fand während zwanzig Stunden bei Raumtemperatur statt.

1.0 ml des gebrauchsfertigen Trennreagenz entsprechend Beispiel 3a), das vor Gebrauch kurz geschüttelt wurde, um eine homogene Zellsuspension zu erhalten, wurde in alle Röhrchen gegeben.

Alle Röhrchen wurden zum Vermischen des Inhalts kurz geschüttelt und zehn bis dreissig Minuten später bei etwa 2000 g zehn Minuten lang zentrifugiert.

Der Niederschlag wurde durch Dekantieren oder Absaugen der überstehenden Flüssigkeit abgetrennt.

Nach dem Abtrennen der überstehenden Flüssigkeit wurde die Gamma-Strahlung des im Niederschlag enthaltenen markierten AFP in allen Röhrchen gemessen.

In einem Diagramm wurden die Impulse der Gamma-Strahlung der Eichproben pro Zeiteinheit linear gegen die AFP-Konzentration der Eichproben aufgetragen. Aus diesem Diagramm konnte dann die AFP-Konzentration der unbekannten Probe bestimmt werden.

Beispiel 4
Bestimmung des humanen Schilddrüsenstimulierenden Hormons (hTSH)
in Blutserum oder Blutplasma
a) Herstellung des Trennreagenzes

Die Herstellung des Trennreagenzes wurde einschliesslich des Waschschrittes entsprechend Beispiel 1a) durchgeführt. Das gebrauchsfertige Trennreagenz wurde hergestellt, indem die so behandelten Zellen mit dem zweiten Antikörper anschliessend in 500 ml einer wässrigen Pufferlösung vom pH 7,4, die 1,3 m Ammonsulfat und 0,05 m Natriumphosphat enthielt, resuspendiert wurden.

b) Durchführung der Bestimmung
0.1 ml der zu untersuchenden humanen Serum- oder Plasmaprobe wurde in ein Röhrchen gegeben.

0.1 ml einer Eichprobe mit verschiedenen hTSH-Konzentrationen, z.B. 0, 6, 12, 25, 50 und 100 $\mu$ Einheiten hTSH/ml wurden jeweils in ein Röhrchen gegeben.

0.1 ml einer $^{125}$J-hTSH enthaltenden Lösung (etwa 0.4 ng hTSH/ml von einer spezifischen Radioaktivität von 80 nCi Jod-125/ng hTSH) wurden in alle Röhrchen gegeben.

0.1 ml einer Antikörperlösung gegen hTSH, die in Abwesenheit von unmarkiertem hTSH 30 bis 50% des $^{125}$J-hTSH binden konnte, wurde in alle Röhrchen gegeben.

Die Inkubation fand während zwanzig Stunden bei Raumtemperatur statt.

0.5 ml des gebrauchsfertigen Trennreagenz entsprechend Beispiel 4a), das vor Gebrauch kurz geschüttelt wurde, um eine homogene Zellsuspension zu erhalten, wurde in alle Röhrchen gegeben.

Alle Röhrchen wurden zum Vermischen des Inhalts kurz geschüttelt und fünfzehn bis dreissig Minuten später bei etwa 2000 g zehn Minuten lang zentrifugiert.

Der Niederschlag wurde durch Dekantieren oder Absaugen der überstehenden Flüssigkeit abgetrennt und mit einer 0.05 m wässrigen Natriumphosphatpufferlösung vom pH 7.4 gewaschen.

Nach dem Abtrennen der überstehenden Flüssigkeit wurde die Gamma-Strahlung des im Niederschlag enthaltenen markierten hTSH in allen Röhrchen gemessen.

In einem Diagramm wurden die Impulse der Gamma-Strahlung der Eichproben pro Zeiteinheit linear gegen die hTSH-Konzentration der Eichproben aufgetragen. Aus diesem Diagramm konnte dann die hTSH-Konzentration der unbekannten Probe 30 bestimmt werden.

Beispiel 5
Bestimmung des humanen Follikelstimulierenden Hormons (hFSH)
in Blutserum oder Blutplasma
a) Herstellung des Trennreagenzes

Die Herstellung des Trennreagenzes wurde einschliesslich des Waschschrittes entsprechend Beispiel 1a) durchgeführt.

Das gebrauchsfertige Trennreagenz wurde hergestellt indem die so behandelten Zellen mit dem zweiten Antikörper anschliessend in 500 ml einer 15%igen Lösung von Polyvinylpyrrolidon mit einem mittleren Molekulargewicht von 10 000 in 0.05 m wässrigem Natriumphosphatpuffer vom pH 7.4 resuspendiert wurden.

b) Durchführung der Bestimmung
Die Analysen wurden in Zentrifugenröhrchen von 3 bis 5 ml aus Polystyrol oder Polypropylen durchgeführt.

0.1 ml der zu untersuchenden humanen Serum- oder Plasmaprobe wurde in ein Röhrchen gegeben.

0.1 ml einer Eichprobe mit verschiedenen hFSH-Konzentrationen, z.B. 0, 5, 10, 20, 40 und 80 m Einheiten hFSH/ml wurden jeweils in ein Röhrchen gegeben.

0.2 ml einer $^{125}$J-hFSH enthaltenden Lösung (etwa 0.5 ng hFSH/ml von einer spezifischen Radioaktivität von 100 nCi Jod-125/ng hFSH) wurden in alle Röhrchen gegeben.

0.2 ml einer Antikörperlösung gegen hFSH, die in Abwesenheit von unmarkiertem hFSH 30 bis 50% des $^{125}$J-hFSH binden konnte, wurde in alle Röhrchen gegeben.

Die Inkubation fand während zwanzig Stunden bei Raumtemperatur statt.

0.5 ml des gebrauchsfertigen Trennreagenz entsprechend Beispiel 5a), das vor Gebrauch kurz geschüttelt wurde, um eine homogene Zellsuspension zu erhalten, wurde in alle Röhrchen gegeben.

Alle Röhrchen wurden zum Vermischen des Inhalts kurz geschüttelt und fünfzehn bis dreissig Minuten später bei etwa 2000 g zehn Minuten lang zentrifugiert.

Der Niederschlag wurde durch Dekantieren oder Absaugen der überstehenden Flüssigkeit abgetrennt und mit einer 0.05 m wässrigen Natriumphosphatpufferlösung vom pH 7.4 gewaschen.

Nach dem Abtrennen der überstehenden Flüssigkeit wurde die Gamma-Strahlung des im Niederschlag enthaltenen markierten hFSH in allen Röhrchen gemessen.

In einem Diagramm wurden die Impulse der Gamma-Strahlung der Eichproben pro Zeiteinheit linear gegen die hFSH-Konzentration der Eichproben aufgetragen. Aus diesem Diagramm konnte dann die hFSH-Konzentration der unbekannten Probe bestimmt werden.

### Patentansprüche

1. Verfahren zum Binden eines an einen ersten Antikörper spezifisch gebundenen Antigens in einer wässrigen Lösung und zum Abtrennen dieses gebundenen Antigens von nicht gebundendem Antigen zur Bestimmung der Antigenkonzentration in einer Körperflüssigkeit mit Hilfe des kompetitiven Radioimmunoassays, wobei der erste Antikörper mit dem spezifisch gebundenen Antigen durch einen zweiten Antikörper, der einerseits an einer Staphylococcus aureus-Zelle gebunden ist und anderseits den ersten Antikörper spezifisch bindet von dem freien, in der wässrigen Lösung gelösten Antigen abgetrennt wird, dadurch gekennzeichnet, dass der wässrigen Lösung ein Reaktionsbeschleuniger zugesetzt wird, der die spezifische Bindung des ersten Antikörpers durch den zweiten Antikörper beschleunigt, wobei als Reaktionsbeschleuniger 4 bis 8 Gewichtsprozent Polyethylenglykol, 5 bis 10 Gewichtsprozent Dextran, 5 bis 10 Gewichtsprozent Polyvinylpyrrolidon, 0,5 bis 0,9 molar Ammoniumsulfat oder 0,8 bis 1,2 molar Natriumsulfit verwendet wird.

2. Reagenz für die Trennung von freiem Antigen von einem an einem ersten Antikörper gebundenen Antigen bei der Bestimmung eines Antigens mit Hilfe des kompetitiven Radioimmunoassays gemäss Anspruch 1, wobei in einer wässrigen Pufferlösung Staphylococcus aureus-Zellen, an die der zweite Antikörper spezifisch gebunden ist, sowie als Reaktionsbeschleuniger Polyethylenglykol, Dextran, Polyvinylpyrrolidon, Ammonsulfat oder Natriumsulfit enthalten sind und zwar in solchen Mengen, dass nach der Zugabe zum Testansatz deren Konzentration 4 bis 8 Gewichtsprozent an Polyethylenglykol, 5 bis 10 Gewichtsprozent an Dextran, 5 bis 10 Gewichtsprozent an Polyvinylpyrrolidon, 0,5 bis 0,9 molar in Bezug auf Ammoniumsulfat oder 0,8 bis 1,2 molar in Bezug auf Natriumsulfit ist.

### Claims

1. Process for binding, in an aqueous solution, an antigen which is specifically bound to a first antibody, and for separating this bound antigen from antigen which is not bound, for the determination of the antigen concentration in a body liquid with the aid of competitive radioimmunoassay, whereby the first antibody with the specifically bound antigen is separated from the free antigen which is dissolved in the aqueous solution, the separation being carried out by means of a second antibody, which is bound, on the one hand, to a Staphylococcus aureus cell, and, on the other hand, specifically binds the first antibody, and the process comprises adding a reaction accelerator to the aqueous solution, the reaction accelerator accelerating the specific binding of the first antibody by the second antibody, whereby 4–8 percent by weight polyethylene glycol, 5–10 percent by weight dextran, 5–10 percent by weight polyvinylpyrrolidone, 0,5–0,9 molar ammonium sulfate or 0,8–1,2 sodium sulfite are used as reaction accelerator.

2. Reagent for the separation of free antigen from an antigen which is bound to a first antibody in the determination of an antigen with the aid of the competitive radioimmunoassay as in claim 1, containing in an aqueous buffer solution Staphylococcus aureus cells to which the second antibody is specifically bound and as reaction accelerator polyethylene glycol, dextran, polyvinylpyrrolidone, ammonium sulfate or sodium sulfite in such amounts that after addition to the test mixture the concentration is 4–8 percent by weight of polyethylene glycol, 5–10 percent by weight of dextran, 5–10 percent by weight of polyvinylpyrrolidone, 0,5–0,9 molar with respect to ammonium sulfate or 0,8–1,2 molar with respect to sodium sulfite.

### Revendications

1. Procédé pour la liaison d'un antigène fixé spécifiquement sur un premier anticorps dans une solution aqueuse et pour la séparation de cet antigène fixé de l'antigène non fixé pour la détermination de la concentration en antigène dans un fluide corporel à l'aide du radioimmunoessai compétitif, où le premier anticorps sur lequel est fixé spécifiquement l'antigène, et séparé de l'antigène libre, dissous dans la solution aqueuse, par un second anticorps qui est d'une part fixé sur une cellule de Staphylococcus aureus et d'autre part lie spécifiquement le premier anticorps, procédé caractérisé en ce que l'on ajoute à la solution aqueuse un accélérateur de réaction qui accélère la liaison spécifique du premier anticorps par les seconds anticorps, en utilisant comme accélérateur de réaction de 4 à 8% en poids de polyéthylène glycol, de 5 à 10% en poids de dextrane, de 5 à 10% en poids de polyvinylpyrrolidone, de 0,5 à 0,9 mole de sulfate d'ammonium ou de 0,8 à 1,2 mole de sulfite de sodium.

2. Réactif pour la séparation d'un antigène libre d'un antigène fixé sur un premier anticorps, pour la détermination d'un antigène à l'aide du radioimmunoessai compétitif selon la revendication 1, où dans une solution tampon aqueuse sont contenues des cellules de Staphylococcus aureus, sur lesquelles est fixé spécifiquement le deuxième anticorps, ainsi qu'un accélérateur de réaction tel que le polyéthylène glycol, le dextrane, la polyvinylpyrrolidone, le sulfate d'ammonium ou le sulfite de sodium, et certes en des quantités telles qu'après l'addition à la préparation d'essai, leur concentration soit de 4 à 8% en poids en polyéthylène glycol, de 5 à 10% en poids en dextrane, de 5 à 10% en poids en polyvinylpyrrolidone, de 0,5 à 0,9 mole de sulfate d'ammonium ou de 0,8 à 1,2 mole de sulfite de sodium.